# EUROPEAN PATENT APPLICATION

(11) **EP 2 211 178 A1**
(43) Date of publication of application: **28.07.2010**
(21) Application number: 09000828.5
(22) Date of filing: 22.01.2009
(51) Int. Cl.: G01N 33/542, G01N 33/58

(54) **Method for detecting an analyte in a sample by multiplexing FRET analysis and kit**

(71) Applicant: Universität Potsdam, 14469 Potsdam (DE); CNRS - Délégation Alsace, 67037 Strasbourg (FR); Cézanne, 30035 Nîmes cedex 1 (FR)
(72) Inventor: Hildebrandt, Niko, Dr., 14169 Berlin (DE); Geissler, Daniel, 14480 Potsdam (DE); Löhmannsröben, Hans-Gerd, Prof.Dr., 14476 Potsdam-Golm (DE); Charbonnière, Loic, Dr., 67720 Weyersheim (FR); Ziessel, Raymond, Dr., 67460 Souffelweyersheim (FR); Bois, Emmanuel, Dr., 30000 Nimes (FR)
(74) Representative: Bittner, Thomas L.

(57) **Abstract**

The invention concerns a method for detecting an analyte in a sample by multiplexing FRET (Förster Resonance Energy Transfer) analysis, the method comprising the following steps: providing a sample containing an energy transfer donor, several spectrally different quantum dot species, and an analyte, wherein the analyte is configured to mediate an energy transfer within a first energy transfer donor-acceptor pair provided by the energy transfer donor and a first quantum dot specie, the energy transfer donor is configured to act as energy transfer donor in the first energy transfer donor-acceptor pair, and the first quantum dot specie is configured to act as energy transfer acceptor in the first energy transfer donor-acceptor pair, irradiating excitation light from an excitation light source to the sample, in the first energy transfer donor-acceptor pair, transferring excitation energy from the energy transfer donor excited by the excitation light to the first quantum dot specie, the energy transfer being mediated by the analyte, and detecting emission light emitted by the first quantum dot specie after receiving the excitation energy.

## Description

The invention refers to a method for detecting an analyte in a sample by multiplexing FRET (Förster Resonance Energy Transfer) analysis, and a kit for detecting one or more analytes in a multiplexing FRET analysis.

### Background of the invention

The theory of FRET (Förster Resonance Energy Transfer) defines a 1/*r*⁶ distance dependent, non-radiative transfer of energy from an excited donor (D) to an acceptor molecule (A). The relationship between easily accessible spectroscopic data and theoretical equations was the achievement of Theodor Förster, thereby enabling the possibility of many FRET applications in all kinds of natural sciences. Details of the theory of FRET are well known.

The bottom line of spectroscopic properties for a successful FRET application is the so called Förster Radius *R*₀, the distance between D and A where the energy transfer is 50 % efficient. *R*₀ can be calculated from spectral data of donor luminescence and acceptor absorption, both relatively easy to measure. It is mainly dependent on the overlap of the luminescence spectrum of the donor and the absorption spectrum of the acceptor as well as the luminescence quantum yield (ratio of emitted to absorbed photons) of the donor. The larger *R*₀ the more efficient is FRET (at a fixed distance) or the larger is the D-A distance (at a fixed FRET efficiency).

FRET has been used in biochemical applications within the 1 to 10 nm scale (K. E. Sapsford et al., Angew. Chem. Int. Ed., 45, 4562, 2006) (e.g. protein-protein binding, protein folding, molecular interactions at and in cell membranes, DNA hybridization and sequencing, immunoreactions of antigens and antibodies). The result of such a FRET measurement is a distance between about 1 and 20 nm or a concentration of a specific substance. As FRET can be analyzed by time-resolved luminescence techniques in solution with myriads of Ds and As, the nanoscale method can become accessible for the macroscopic world. Moreover, confocal microscopic techniques can analyze FRET on a single-molecule level in order to measure parameter distributions rather than averaging over an ensemble.

To date *R*₀ values for commonly used D-A pairs are rarely larger than 6 nm (only 4 out of 273 FRET pairs with *R*₀ between 6 and 7 nm (B. W. Van der Meer et al., Resoncance Energy Transfer: Theory and Data, Wiley-VCH, New York). Combining lanthanide complexes (LCs) as donors and quantum dots as acceptors in a biochemical FRET application Förster radii of more than 10 nm can be found. Thus breaking the common limit of 10 nm is possible. FRET is relatively well understood for LCs (P. R. Selvin, Ann. Rev. Biophys. Biomol. Struct., 31, 275, 2002) but a profound understanding of QD-based FRET (especially with QDs as As) is lacking.

Quantum dots (QDs) are well characterized and established nanoparticles (especially CdSe/ZnS core/shell dots) with unique optical and photophysical properties. QDs are composed of a nanometer-sized core of a semiconductor material, often coated by a passivating shell consisting of a larger bandgap semiconductor, and an external coating shell for water solubility and biocompatability. Depending on both the semiconductor material and the size of the nanocrystals, (I. L. Medintz et al., Nat. Mater., 4, 435, 2005) the absorption and emission properties can be tuned in almost all of the spectral domain from UV to near infrared. QDs display extremely large one- and two-photon absorption cross sections, the possibility of large spectral separations between excitation and emission, and narrow emission bands. QDs are also commercially available (e.g. Invitrogen, Evident Technologies).

QDs often display excellent luminescence quantum yields and are far more resistant to photo-bleaching than organic dyes (X. Y. Wu, Nat. Biotechnol. 2003, 21, 41). In addition, many Ds or As can be attached to the large surface of a single QD thus allowing for multiple-molecule FRET with increased overall efficiency.

It was shown that QDs can hardly be efficient As when combined with common organic fluorophores as Ds possibly due to the short-lived excited states (ns) of these fluorophores (A. R. Clapp et al., J. Am. Chem. Soc., 127 1242, 2005). Contrary to these results, in a recent publication Zhao et al. claimed FRET from FITC (FITC = fluorescein isothiocyanate) to CdSe/ZnS core/shell QDs (J. H. Wang et al., Colloids Surf. A, 302, 168, 2007). Unfortunately, there is little spectroscopic data available (steady-state spectra only) to confirm this FRET so it is very questionable if this is real FRET. Other contributions deal with QDs both as Ds and As e.g. Y. B. Li et al., Luminescence, 22, 60, 2007. However, the drawback of short-lived excited states can be overcome by using LCs as Ds, which usually possess very long-lived luminescence (up to ms). Besides applications dealing with bioluminescence resonance energy transfer (BRET), an efficient possibility of energy transfer to QDs was found to involve LCs as Ds ( a) L. J. Charbonnière et al., J. Am. Chem. Soc., 128, 12800, b) 2006, N. Hildebrandt et al., Angew. Chem. Int. Ed., 44, 7612, 2005, c) N. Hildebrandt et al., J. Biomed. Biotechnol., Article ID 79169, 6 pages, 2007, d) H. Härmä et al., Anal Chim. Acta, 604, 177, 2008; e) N. Hildebrandt, Dissertation Thesis, University of Potsdam (Potsdam), 2006; f) N. Hildebrandt et al., Curr. Chem. Biol., 1, 167, 2007.)

A profound understanding of the FRET process by detailed investigations of energy transfer from and to QDs (used as Ds and As) is to date not available. This means that it is not yet clear whether the energy transfer from the LCs to the QDs is real Förster type energy transfer. Another uncertainty is related to size and stability. Large protective shells around the QD result in good stability but large distances between D and A (FRET becomes less efficient). Passing on protective shells leads to small D-A distances but less stability. Moreover, the protective shells have influence on luminescence quantum yields leading to another uncertainty when changing from one QD to another within a FRET system.

FRET has been used as a multiplexing analysis as well. The word multiplexing describes the measurement of several parameters within one and the same sample, e.g. the simultaneous detection of several tumor markers within one blood/serum/plasma sample of a patient. To date optical multiplexing FRET measurements with QDs have only been achieved using QDs as FRET donors. In a recent study (A. R. Clapp et al., J. Am. Chem. Soc. 2005, 127, 18212) a series of experiments was designed analyzing the use of QDs within the frame of multiplexed FRET systems. By using single QD donors associated to two energy acceptors (a fluorescent Cy3 and a nonfluorescent QSY-7) and a set of up to four QD donors (emitting at 510, 555, 570, and 590 nm, respectively) associated to Cy3 or QSY-7, they critically quantified and analyzed the multiplexed systems.

It was also demonstrated that two different QDs emitting at 605 and 655 nm, respectively, can be used with the same LC donor within the same bioassay type, which is binding of biotin (attached on the surface of QDs) to streptavidin (labeled with the LCs). These results were achieved in separated samples. It provides a demonstration that two different QDs can be used as FRET acceptors within a bioassay.

### Summary of the invention

It is the object of the invention to provide an improved method for detecting an analyte in a sample by multiplexing FRET (Förster Resonance Energy Transfer) analysis, and a kit for optically detecting one or more analytes in a multiplexing FRET analysis.

According to the invention a method for optical detection of an analyte in a sample by multiplexing FRET analysis as defined in claim 1 and a kit as defined in claim 13 are provided. Advantageous improvements of the invention are provided in the dependent claims.

According to one aspect of the invention, a method for detecting an analyte in a sample by multiplexing FRET (Förster Resonance Energy Transfer) analysis is provided, the method comprising the following steps:
- providing a sample containing an energy transfer donor, several spectrally different quantum dot species, and an analyte, wherein:
   - the analyte is configured to mediate an energy transfer within a first energy transfer donor-acceptor pair provided by the energy transfer donor and a first quantum dot specie,
   - the energy transfer donor is configured to act as energy transfer donor in the first energy transfer donor-acceptor pair, and
   - the first quantum dot specie is configured to act as energy transfer acceptor in the first energy transfer donor-acceptor pair,
- irradiating excitation light from an excitation light source to the sample,
- in the first energy transfer donor-acceptor pair, transferring excitation energy from the energy transfer donor excited by the excitation light to the first quantum dot specie, the energy transfer being mediated by the analyte, and
- detecting emission light emitted by the first quantum dot specie after receiving the excitation energy.

According to another aspect of the invention, a kit for detecting one or more analytes in a multiplexing FRET analysis is provided, especially for use in a method according to at least one of the preceding claims, the kit comprising several spectrally different quantum dot species and at least one energy transfer donor, wherein the several spectrally different quantum dot species and the at least one energy transfer donor are configured to provide one or more energy transfer donor-acceptor pairs in a sample comprising the one or more analytes, and wherein upon light excitation of the at least one energy donor energy transfer is mediated by the one or more analytes in the one or more energy transfer donor-acceptors pairs.

The detection of the emission light may be done by at least one of steady-state and time-resolved measurements.

The invention provides the use of more than one quantum dot (QD) as an energy transfer (ET) acceptor in one and the same sample (multiplexing) for analyte detection, especially for biological and biochemical applications. The measurement of different analytes within one sample is of special interest due to economic reasons such as less time, less space, less sample preparation, less sample constituent volume all leading to saving time and money. Important fields such as point-of-care and high-throughput screening can significantly profit from the invention.

In one embodiment, lanthanide complexes (LCs) with long luminescence lifetimes are used as ET donors. Other molecules or particles are also possible ET donors. Simultaneously several different QDs, e.g. up to five or more different QDs, are provided within one sample.

The invention can be used for all kinds of measurements in spectroscopy and microscopy, preferably in cases where energy transfer distances of about 1 nm to 20 nm play a role. A quantitative measurement could be e.g. an immunoassay where donor and acceptor are labeled to different antibodies, aptamers, antigens or proteins and the concentration (quantitative signal) of the marker protein, e.g. tumor markers, has to be measured. A qualitative measurement could be e.g. the distance measurement within protein folding, within RNA or DNA based complexes or within cell-based measurements, e.g. the investigation of signal transduction in cells. This ET distance measurement within the range of 1 nm to 20 nm is sometimes referred to as "spectroscopic ruler".

In an embodiment, wherein the method further comprises steps of:
- providing the sample with an additional analyte which is different from the analyte, wherein:
   - the additional analyte is configured to mediate an energy transfer within a second energy transfer donor-acceptor pair provided by the energy transfer donor and a second quantum dot specie which is different from the first quantum dot specie,
   - the energy transfer donor is configured to act as energy transfer donor in the second energy transfer donor-acceptor pair, and
   - the second quantum dot specie is configured to act as energy transfer acceptor in the second energy transfer donor-acceptor pair,
- in the second energy transfer donor-acceptor pair, transferring excitation energy from the energy transfer donor excited by the excitation light to the second quantum dot specie, wherein the energy transfer is mediated by the additional analyte, and
- detecting emission light emitted by the second quantum dot specie after receiving the excitation energy, the emission light emitted by the second quantum dot specie being spectrally different from the emission light emitted by the first quantum dot specie.

In a further embodiment, the method further comprises steps of:
- providing the sample with a further energy transfer donor which is different from the energy transfer donor, wherein:
   - the analyte is configured to mediate an energy transfer within a further energy transfer donor-acceptor pair provided by the further energy transfer donor and one of the first, the second and a third quantum dot specie,
   - the further energy transfer donor is configured to act as energy transfer donor in the further energy transfer donor-acceptor pair, and
   - the first, the second or the third quantum dot specie is configured to act as energy transfer acceptor in the further energy transfer donor-acceptor pair,
- in the further energy transfer donor-acceptor pair, transferring excitation energy from the further energy transfer donor excited by the excitation light to one of the first, the second and the third quantum dot specie, wherein the energy transfer is mediated by the analyte, and
- detecting emission light emitted by one of the first, the second and the third quantum dot species after receiving the excitation energy, the emission light being spectrally different from the emission light emitted by the first quantum dot specie in the first energy transfer donor-acceptor pair and the emission light emitted by the second quantum dot specie in the second energy transfer donor-acceptor pair.
The third quantum dot specie is different from the first and the second quantum dot species.

In a further embodiment, the method further comprises steps of simultaneously detecting at least two of the emission light emitted by the first quantum dot specie in the first energy transfer donor-acceptor pair, the emission light emitted by the second quantum dot specie in the second energy transfer donor-acceptor pair, and the emission light emitted by the one of the first, the second and the third quantum dot species in the further energy transfer donor-acceptor pair.

In still a further embodiment, the method further comprises a step of providing the sample with at least three different analytes, each of the analytes being configured to selectively mediate energy transfer in energy transfer donor-acceptor pairs in the sample.

In another embodiment, the method further comprises a step of deriving structural information from the detected emission light.

In a further embodiment, the step of deriving structural information comprises a step of determining an energy transfer donor-acceptor distance for the excitation energy transfer in at least one of the first energy transfer donor-acceptor pair, the second energy transfer donor-acceptor pair, and the further energy transfer donor-acceptor pair.

In still a further embodiment, the step of determining the energy transfer donor-acceptor distance for excitation energy transfer comprises a step of determining an energy transfer donor-acceptor distance between about 1 nm and about 20 nm.

In a further embodiment, the step of determining the energy transfer donor-acceptor distance for excitation energy transfer comprises a step of determining an energy transfer donor-acceptor distance in a structure selected from the following group of structures: chemical structure, biochemical structure, and biological structure such as DNA or RNA structure, protein folding structure or cell structure.

In a further embodiment, the method further comprises a step of deriving concentration information from the detected emission light.

In another further embodiment, the method further comprises a step of providing the sample as a labeled sample in which the several spectrally different quantum dot species are labeled to different labeling species selected from the following group of labeling species: chemical structure and a biomolecule such as antibody, aptamer, antigen, protein, hormone, DNA, RNA, cell or virus.

In still another embodiment, the method further comprises a step of detecting emission light emitted by at least one of the energy transfer donor and the further energy transfer donor. The detection of the emission light may be done by at least one of steady-state and time-resolved measurements. The analysis of the energy transfer donor emission can be used, for example, to derive further concentration and / or structure information or to affirm information found through energy transfer acceptor emission analysis. The analysis of the energy transfer donor emission can also be used for a correction of fluctuation effects in the sample, e.g. concentration fluctuation of sample constituents or disturbing compounds in the sample, or the excitation and detection setup, e.g. light intensity fluctuations, disturbing background emission, possibly leading to higher sensitivity, lower detection limits and better accuracy and reproducibility.

### Description of preferred embodiments of the invention

Following the invention will be described in further detail, by way of example, with reference to different embodiments. In the figures:
- Fig. 1: shows a schematic representation of the principle of multiplexed energy trans- fer (ET) bioassay using quantum dots (QDs) as acceptors,
- Fig. 2: shows a schematic representation of homogeneous immunoassays principle with several antibodies or aptamers (1-X a/b - specific to marker proteins 1-X) labeled with one donor (D) and several acceptors (A1-AX) for color multiplex ing,
- Fig. 3: shows spectroscopic ruler measurements in which the distance between a do nor (D) and an acceptor (A) is of importance,
- Fig. 4: shows a schematic representation of the binding of the protein streptavidin to the quantum dots,
- Fig. 5: shows emission spectra of Tb lanthanide complex (LC) and the five QDs,
- Fig. 6: shows spectroscopic data of the different QDs, when used as acceptor with the Tb complex as acceptor, and
- Fig. 7A to 7E: show a representation of the intensity ratios (intensity of the QD donor divided by the intensity of the Tb acceptor within a time window of 100 - 1200 µs) of five QDs measured at the different emission wavelengths of the QDs.

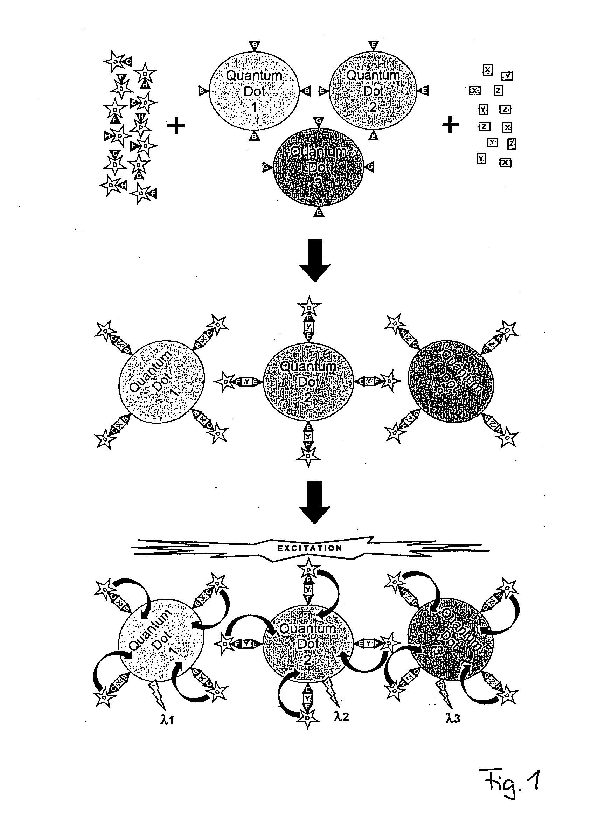

Fig. 1 schematically represents the principle of multiplexed energy transfer (ET) bioassay using quantum dots (QDs) as acceptors. The principle shows the example of multiplexing with the number of QDs n=3 (triplexing), which is representative for several different QDs (n>1). In the top picture 3 different QDs (different emission wavelengths) are labeled with different biomolecules (number of biomolecules m≥1, here 4) B, E and G (e.g. antibodies, proteins, RNA, DNA etc.). These biomolecules can bind to other molecules (here X, Y and Z), which can consist of a number of t≥0 (for p=0 B would directly bind to C, E to F and G to H; for p≥1 X, Y and Z would consist of a complex of different molecules).

The counterpart biomolecules (here C, F and H) are bound to a luminescent ET donor (D), e.g. a lanthanide complex (LC). These donors can be of the same (e.g. D is always the same LC) or of different kind (e.g. different LCs). Once the different molecule complexes (left, center and right in the top picture) are mixed together in one sample they can bind each other as shown in the middle picture. This binding leads to a proximity of donor, e.g. LC, and acceptor (QDs) in the range of about 1 nm to about 20 nm and ET (curved black arrows) becomes possible after excitation of the donors (bottom picture). This ET leads to an excitation of the QDs followed by emission of the different QDs with the different wavelengths.

Time-resolved measurement of the emission of donors and acceptors leads to a distinction between emission caused by ET or emission through direct excitation. Each time-dependent ET emission signal (of different color) is caused by the specific complex between donor and acceptor. In this example QD1 emission gives a quantitative signal of the complex BCX and a qualitative signal about the length of that complex, QD2 gives the same for EYF and QD3 for GZH. Thus concentrations of molecules (e.g. biomolecules such as antigens, proteins etc.) can be measured as well as distances over a range of 1 to 20 nm.

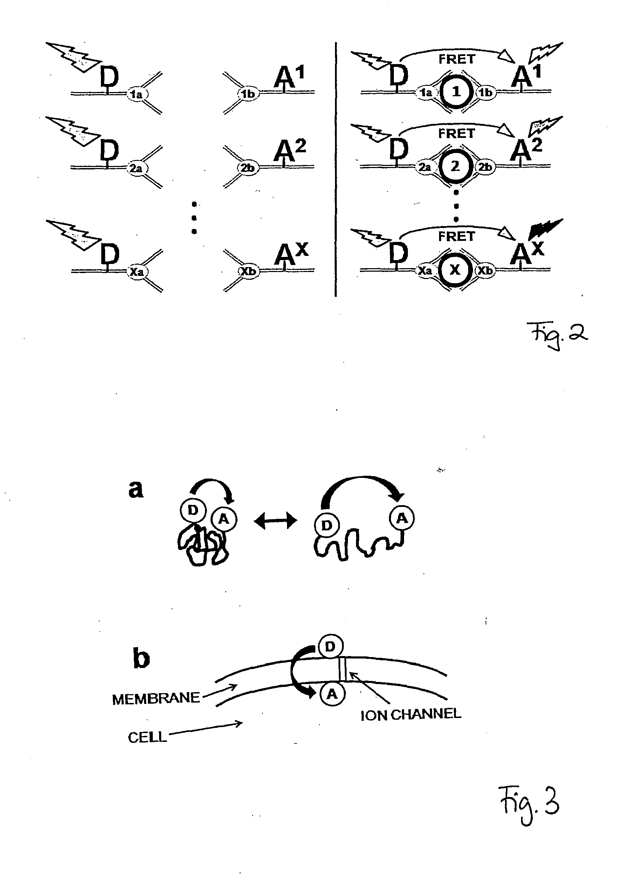

Fig. 2 schematically represents homogeneous immunoassays principle with several antibodies or aptamers (1-X a/b - specific to marker proteins 1-X) labeled with one donor (D) and several acceptors (A1-AX) for color multiplexing. Without protein (left in Fig. 2) markers only D exhibits a long-lived luminescence signal after pulsed light excitation (large D-A distance → no FRET). Addition of different proteins (right in Fig. 2) leads to specific binding and concomitant specific (color) long-lived luminescence signals from A1-AX (small D-A distance → FRET).

It is not obligatory that the immunoassay consists of a sandwich complex "Xa-X-Xb". It is also possible and necessary for some applications (e.g. when the marker protein can only bind one antibody or aptamer) that X is directly labeled with donor or acceptor and a competitive assay (with labeled and non-labeled X) is performed.

Fig. 3 shows spectroscopic ruler measurements in which the distance between a donor (D) and an acceptor (A) is of importance. The black curved arrows represent FRET. a) The FRET signal is a measure for the distance between D and A. Thus the distances within a protein (black curve) folding mechanism can be investigated in detail. There will be less signal the further D and A are apart. b) Signal transduction in cells is of great importance. FRET can be used e.g. to measure interaction in ion channels.

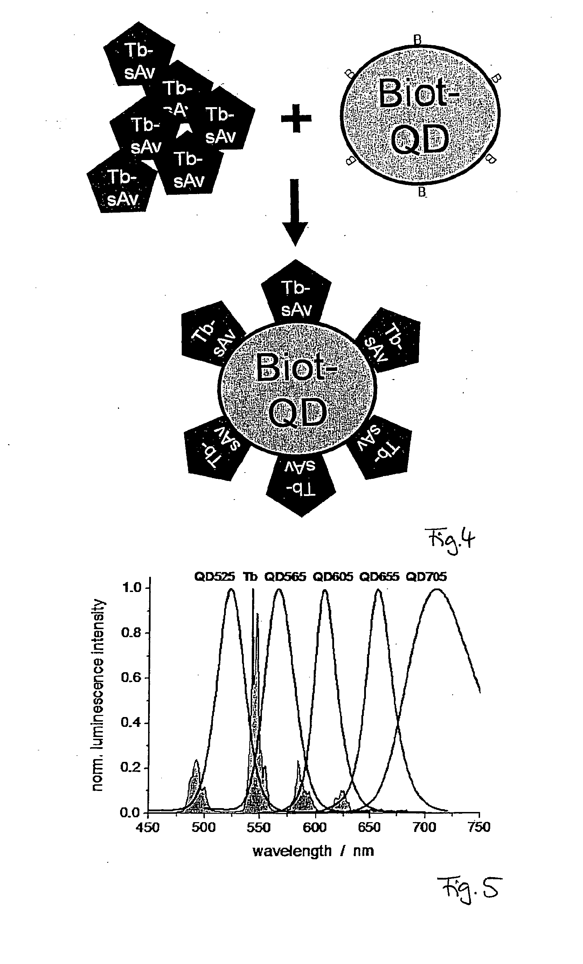

Fig. 4 schematically represents the binding of the protein streptavidin to quantum dots. Once the protein streptavidin (sAV), which, in the present embodiment, is labeled with approximately 12 Tb LCs, is mixed in solution with biotinylated (B) QDs the strong biotin-streptavidin binding forms a donor-acceptor complex and FRET from Tb to QD becomes possible. In general, labeling with one or more Tb LCs is possible.

In order to experimentally demonstrate the multiplexing with quantum dots (QDs) used as FRET acceptors five different QDs with emission wavelengths of 525, 565, 605, 655 and 705 nm labeled with about 3 to 6 biotin molecules on the surface were used. All QDs except QD705 which is of CdSeTe/ZnS type were CdSe/ZnS core/shell type QDs with a further polymer shell and a biocompatibility shell around the core/shell structure. These biotinylated QDs were mixed with streptavidin which was labeled with about 12 Tb complexes. The strong binding of biotin and streptavidin brings the Tb-LCs (as donors) and the QDs (as acceptors) in close proximity (cf. Fig. 4), enabling FRET from Tb to the different QDs.

After excitation with pulsed UV light at 315 nm the Tb and all QDs show a long-lived luminescence, which are the different FRET signals. Fig. 5 shows emission spectra of the Tb LC and the five QDs. All the emission is simultaneous present within the multiplexing assay. Emission intensity is normalized to unity for all emission peaks.

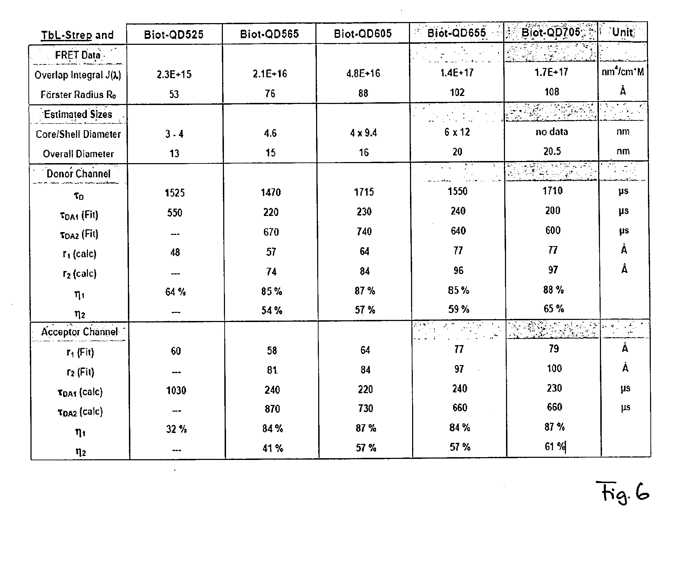

The important spectroscopic data of the different QDs, when used as acceptor with the Tb complex as acceptor are shown in Table in Fig. 6.

In Table 1 multiplexed characterization of distances with QDs used as acceptors and one TbLC as acceptor in a biotin-streptavidin bioassay is shown. FRET data shows the spectral overlap of donor emission and acceptor absorption and the resulting Förster radii. The estimated sizes are given by the supplier (Invitrogen). The time-resolved emission is analyzed for Tb (donor channel) and each QD. τ are luminescence decay times in absence of FRET (τ_{D}) and in presence of FRET (τ_{DA}). η are the FRET efficiencies and r represent the D-A distance. As there are many Tb-LCs labeled to streptavidin and the dots are not all spherical but also ellipsoidal there are several possible distances. For calculation reasons two average distances were chosen, that represent all the other possible distances. It can be seen that the calculated distances fit quite well the estimated sizes and the models of the different QDs, which manily influence the D-A distances.

It can be seen, that the Förster radii, the sizes and the geometry (spherical or ellipsoidal) of the QDs are very different. Time-resolved analysis of donor emission and acceptor emission, respectively, show that different distances between D and A due to the difference in QD size can be measured (the D-A distance is increasing with increasing size of the QDs). This clearly demonstrates that the invention can measure distances within a multiplexing assay (multiplexed spectroscopic ruler).

In order to demonstrate a sensitive quantitative analysis, small volumes of 1nM Biot-QDs were added to 1nM of Tb-sAv and the time gated long-lived emission (between 0.1 and 1.2 ms) was measured on a modified commercial immunoanalyzer (KRYPTOR of Cezanne and BRAHMS). The addition leads to binding and results in FRET from Tb to the QDs and concomitant long-lived QD emission. All Biot-QDs could be detected with sub-picomolar detection limits within one and the same sample. Control experiments were performed with pure Tb-LC to show that the emission signals are caused by FRET due to the streptavidin-biotin binding and not due to diffusion within the solution.

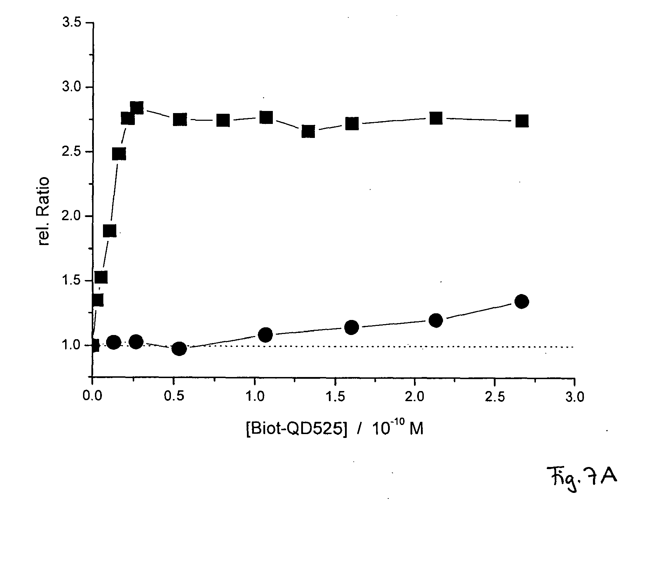

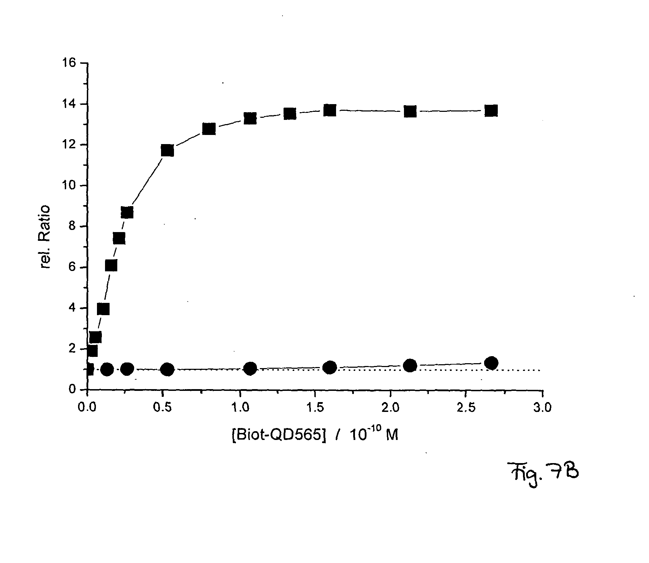

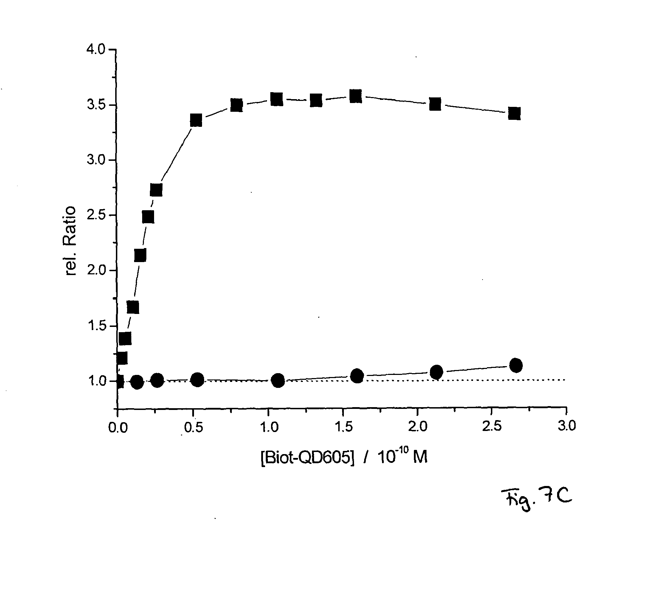

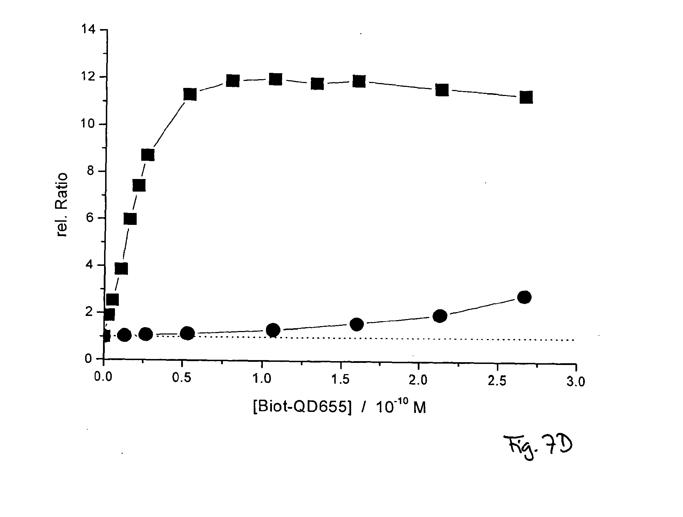

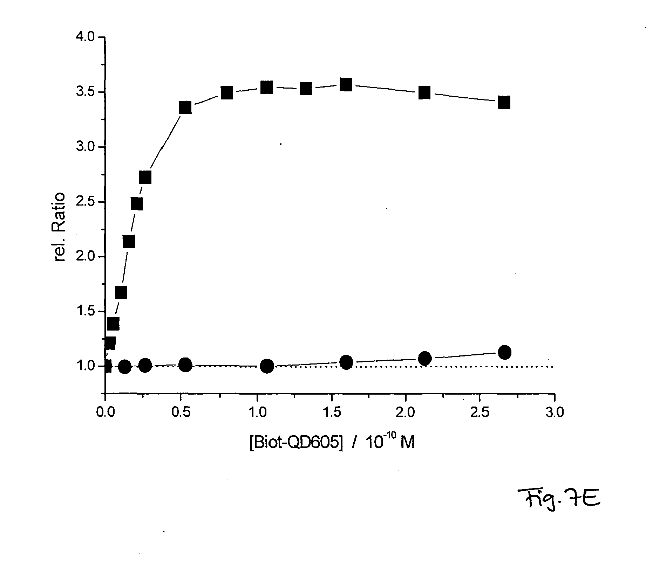

Fig. 7A to 7E show the intensity ratios (intensity of the QD donor divided by the intensity of the Tb acceptor within a time window of 100 - 1200 µs) of all five QDs measured at the different emission wavelengths of the QDs. The squares show the strong increase of the relative ratio (normalized to unity at zero concentration) due to the binding of biotin and streptavidin and the concomitant ET from Tb to the different QDs. The dots show the control experiments where Tb was used without streptavidin. The ratio increase cannot be found here which demonstrates that the ET is enabled by the binding of biotin and streptavidin in the bioassay. The limits of detection (3-fold standard deviation of 30 measurements at zero concentration divided by the slope of the linear part of the increasing ratio curve) are all sub-picomolar within the same sample, showing the high sensitivity of the multiplexed bioassay with QDs as acceptors.

The features disclosed in at least one of the specification, the claims and the figures may be material for the realization of the invention in its various embodiments, taken in isolation or in various combinations thereof.

## Claims

1. A method for detecting an analyte in a sample by multiplexing FRET (Förster Resonance Energy Transfer) analysis, the method comprising the following steps:
- providing a sample containing an energy transfer donor, several spectrally different quantum dot species, and an analyte, wherein:
- the analyte is configured to mediate an energy transfer within a first energy transfer donor-acceptor pair provided by the energy transfer donor and a first quantum dot specie,
- the energy transfer donor is configured to act as energy transfer donor in the first energy transfer donor-acceptor pair, and
- the first quantum dot specie is configured to act as energy transfer acceptor in the first energy transfer donor-acceptor pair,
- irradiating excitation light from an excitation light source to the sample,
- in the first energy transfer donor-acceptor pair, transferring excitation energy from the energy transfer donor excited by the excitation light to the first quantum dot specie, the energy transfer being mediated by the analyte, and
- detecting emission light emitted by the first quantum dot specie after receiving the excitation energy.

2. Method according to claim 1, wherein the method further comprises steps of:
- providing the sample with an additional analyte which is different from the analyte, wherein:
- the additional analyte is configured to mediate an energy transfer within a second energy transfer donor-acceptor pair provided by the energy transfer donor and a second quantum dot specie which is different from the first quantum dot specie,
- the energy transfer donor is configured to act as energy transfer donor in the second energy transfer donor-acceptor pair, and
- the second quantum dot specie is configured to act as energy transfer acceptor in the second energy transfer donor-acceptor pair,
- in the second energy transfer donor-acceptor pair, transferring excitation energy from the energy transfer donor excited by the excitation light to the second quantum dot specie, wherein the energy transfer is mediated by the additional analyte, and
- detecting emission light emitted by the second quantum dot specie after receiving the excitation energy, the emission light emitted by the second quantum dot specie being spectrally different from the emission light emitted by the first quantum dot specie.

3. Method according to claim 1 or 2, wherein the method further comprises steps of:
- providing the sample with a further energy transfer donor which is different from the energy transfer donor, wherein:
- the analyte is configured to mediate an energy transfer within a further energy transfer donor-acceptor pair provided by the further energy transfer donor and one of the first, the second and a third quantum dot specie,
- the further energy transfer donor is configured to act as energy transfer donor in the further energy transfer donor-acceptor pair, and
- the first, the second or the third quantum dot specie is configured to act as energy transfer acceptor in the further energy transfer donor-acceptor pair,
- in the further energy transfer donor-acceptor pair, transferring excitation energy from the further energy transfer donor excited by the excitation light to one of the first, the second and the third quantum dot specie, wherein the energy transfer is mediated by the analyte, and
- detecting emission light emitted by one of the first, the second and the third quantum dot species after receiving the excitation energy, the emission light being spectrally different from the emission light emitted by the first quantum dot specie in the first energy transfer donor-acceptor pair and the emission light emitted by the second quantum dot specie in the second energy transfer donor-acceptor pair.

4. Method according to claim 2 or 3, wherein the method further comprises steps of simultaneously detecting at least two of:
- the emission light emitted by the first quantum dot specie in the first energy transfer donor-acceptor pair,
- the emission light emitted by the second quantum dot specie in the second energy transfer donor-acceptor pair, and
- the emission light emitted by the one of the first, the second and the third quantum dot species in the further energy transfer donor-acceptor pair.

5. Method according to one of the preceding claims, wherein the method further comprises a step of providing the sample with at least three different analytes, each of the analytes being configured to selectively mediate energy transfer in energy transfer donor-acceptor pairs in the sample.

6. Method according to one of the preceding claims, wherein the method further comprises a step of deriving structural information from the detected emission light.

7. Method according to claim 6, wherein the step of deriving structural information comprises a step of determining an energy transfer donor-acceptor distance for the excitation energy transfer in at least one of the first energy transfer donor-acceptor pair, the second energy transfer donor-acceptor pair, and the further energy transfer donor-acceptor pair.

8. Method according to claim 7, wherein the step of determining the energy transfer donor-acceptor distance for excitation energy transfer comprises a step of determining an energy transfer donor-acceptor distance between about 1 nm and about 20 nm.

9. Method according to claim 7 or 8, wherein the step of determining the energy transfer donor-acceptor distance for excitation energy transfer comprises a step of determining an energy transfer donor-acceptor distance in a structure selected from the following group of structures: chemical structure, biochemical structure, and biological structure such as DNA or RNA structure, protein folding structure or cell structure.

10. Method according to one of the preceding claims, wherein the method further comprises a step of deriving concentration information from the detected emission light.

11. Method according to one of the preceding claims, wherein the method further comprises a step of providing the sample as a labeled sample in which the several spectrally different quantum dot species are labeled to different labeling species selected from the following group of labeling species: chemical structure and a biomolecule such as antibody, aptamer, antigen, protein, hormone, DNA, RNA, cell or virus.

12. Method according to one of the preceding claims, wherein the method further comprises a step of detecting emission light emitted by at least one of the energy transfer donor and the further energy transfer donor.

13. Kit for detecting one or more analytes in a multiplexing FRET analysis, especially for use in a method according to at least one of the preceding claims, the kit comprising several spectrally different quantum dot species and at least one energy transfer donor, wherein the several spectrally different quantum dot species and the at least one energy transfer donor are configured to provide one or more energy transfer donor-acceptor pairs in a sample comprising the one or more analytes, and wherein upon light excitation of the at least one energy donor energy transfer is mediated by the one or more analytes in the one or more energy transfer donor-acceptors pairs.

14. Kit according to claim 13, wherein the kit is provided as an immunoassay type kit.

15. Kit according to claim 12 or 13, wherein the kit is provided as a distance measurement kit.
